# EUROPEAN PATENT SPECIFICATION

(11) **EP 4 488 671 B1**
(45) Date of publication and mention of the grant of the patent: **25.02.2026**
(21) Application number: 23183778.2
(22) Date of filing: 06.07.2023
(51) Int. Cl.: G01N 27/404, G01N 27/416, G01N 33/00, G01N 27/403, G01N 33/18, G01N 27/30

(54) **AMMONIUM ANALYZER ARRANGEMENT**
AMMONIUMANALYSATORANORDNUNG
DISPOSITIF D'ANALYSE D'AMMONIUM

(43) Date of publication of application: 08.01.2025
(73) Proprietor: Hach Lange GmbH, 14163 Berlin (DE)
(72) Inventor: RUDDE, Heinz, 14163 Berlin (DE); STELLMACH-HANULOK, Aurelia, Berlin 14163 (DE); HAHN, Markus, 14163 Berlin (DE)
(74) Representative: terpatent PartGmbB

(56) References cited:
- JP-A- 2010 256 085
- US-A1- 2006 070 889
- US-A1- 2018 180 574
- US-A1- 2022 050 077
- US-B2- 11 307 167

## Description

The invention refers to an ammonium analyzer arrangement with a gas-selective electrode unit with an electrolyte chamber housing filled with an electrolyte liquid and refers to a method for determining the actual volume of the electrolyte liquid in the electrolyte chamber housing of the gas-selective electrode unit.

A typical ammonium analyzer arrangement is known from US 2018 180 574 A1, US 2022 050 077 A1, JP 2010 256 085 A and US 11 307 167 B2.

US 2022/050077 A1 discloses a measuring device configured to measure a state of a solution. The remaining amount of the inner solution is measured based on any sensor installed inside the support.

Such typical ammonium analyzer arrangements are provided with a gas-selective electrode unit immersed into a sample chamber which is filled with a chamber liquid being a sample liquid or a calibration liquid. The gas-selective electrode unit is provided with an electrolyte chamber housing filled with an electrolyte liquid and is provided with a measurement electrode element being in direct contact with the electrolyte liquid. The electrolyte chamber housing is provided with a gassensitive membrane separating the electrolyte liquid within the electrolyte chamber housing from the chamber liquid in the sample chamber.

Typically, the gas-sensitive membrane is very thin and mechanically sensitive to a pressure difference over the gas-sensitive membrane. Therefore, the electrolyte chamber housing is provided with a venting opening so that the gas cushion above the electrolyte liquid is always at constant atmospheric pressure, as disclosed in US 2006 0 07 0 889 A1. However, the electrolyte liquid constantly evaporates through the venting opening so that the electrolyte liquid volume of the electrolyte liquid within the electrolyte chamber housing constantly decreases.

For guaranteeing constant conditions and allowing precise determinations of the ammonium concentration, the gas-selective electrode unit is kept at a constant temperature at, for example, 45°C so that the evaporation of the electrolyte liquid is relatively high.

As a consequence, the ammonium analyzer arrangement must be periodically calibrated at, for example, a daily basis. Another consequence of the constant evaporation of the electrolyte liquid is the fact, that the electrolyte liquid is finally exhausted. However, it is difficult to determine the electrolyte volume with a suitable sensor. In practice, the electrolyte chamber housing is refilled with the electrolyte liquid in constant maintenance intervals of, for example, 90 days.

It is an object of the invention to provide an ammonium analyzer arrangement with a gas-selective electrode unit and a method for providing a high reliability and a long maintenance interval of the gas-selective electrode unit.

This object is, according to the invention, solved with an ammonium analyzer arrangement with the features of claim 1 and with a method for determining the actual volume of an electrolyte liquid in an ammonium analyzer arrangement with the features of method claim 7.

The ammonium analyzer arrangement according to the invention is provided with an electronic controller and with a measurement unit which is controlled by the electronic controller. The ammonium analyzer arrangement can be a laboratory device, but preferably is a process device with maintenance intervals which should be as long as possible.

The measurement unit comprises a sample chamber which is filled with a chamber liquid which is, in addition to a reagent liquid, either a sample liquid from a sample liquid site, for example from a water treatment plant basin, or is filled with a calibration liquid for performing a calibration run.

The measurement unit also comprises a gas-selective electrode unit with an electrolyte chamber housing filled with a volume of an electrolyte liquid. The electrolyte chamber housing is not perfectly fluid-tight but is provided with a venting opening which guarantees that the gas pressure of the gas cushion within the electrolyte chamber housing is always at atmospheric pressure.

The gas-selective electrode unit comprises a reference electrode element which is in direct contact with the electrolyte liquid. The reference electrode element can be of any suitable type. The reference electrode element is electrically connected to a measurement signal evaluation module.

The electrolyte chamber housing is provided with a gas-sensitive membrane directly separating the electrolyte liquid within the electrolyte chamber housing from the chamber liquid in the sample chamber. The gassensitive membrane can be of any suitable material, for example can be made of silicone. Preferably, the gas-sensitive membrane is made of PTFE (Polytetrafluoroethylene).

The gas-selective electrode unit is also provided with a separate measurement electrode element being in direct contact with the electrolyte liquid within the electrolyte chamber housing. The gas-selective electrode unit is, at least in part, always immersed into the chamber liquid.

Preferably, the sensing surface of the measurement electrode element is provided very close to the gas-sensitive membrane but is not in direct contact with the gas-sensitive membrane so that a thin layer of the electrolyte liquid is provided between the sensing surface of the measurement electrode element and the gas-sensitive membrane. More preferably, this thin electrolyte liquid layer has a volume of less than 1 ml.

The ammonium analyzer arrangement is provided with a calibration liquid container with a calibration liquid which is pumped from the calibration liquid container to the sample chamber, if a calibration run is initiated, for example at a daily basis. Preferably, the calibration liquid has an ammonium standard concentration of at least 5 mg/I.

The electronic controller comprises a measurement signal evaluation module which is electrically connected to the reference electrode element and to the gas-selective electrode unit for generating a differential measurement value.

The electronic controller also comprises a calibration module generating a calibration liquid measurement value when the sample chamber is filled with the calibration liquid. The calibration liquid measurement value is important for the measurement signal evaluation module for providing a correct measurement value. The measurement signal evaluation module generates as the measurement value an electric voltage typically in the range of -300 mV to +300 mV for a measurement range of 0 to 1000 mg/l ammonium (NH4-N).

The electronic controller is also provided with an electrolyte exhaustion determination module comprising an exhaustion-graph-memory memorizing a calibration/exhaustion graph. The calibration/exhaustion graph describes a relation of the calibration liquid measurement value versus the absolute actual volume of the electrolyte liquid in the gas-selective electrode unit, or describes another relation of the calibration liquid measurement value and the actual volume of the electrolyte liquid in the gas-selective electrode unit. Typically, the calibration liquid measurement value increases with a decreasing volume of the electrolyte liquid in the electrolyte chamber housing.

The electrolyte exhaustion determination module is in signal-connection with the calibration module. When a calibration run has been made, the electrolyte exhaustion determination module determines the actual volume of the electrolyte liquid in the electrolyte chamber housing at the basis of the actual calibration measurement value and the calibration/exhaustion graph. The electronic controller can process the determined actual volume of the electrolyte liquid in different ways, for example, can send a warning signal when the electrolyte liquid volume has reached a minimum volume so that the electrolyte chamber housing needs to be refilled with electrolyte liquid. As a result, there is no need for a fixed maintenance interval with an appropriate safety margin, so that, in practice, the maintenance interval for refilling the electrolyte chamber housing with the electrolyte liquid can be extended from 90 up to 180 days.

Preferably, the measurement unit is provided with a heating and a temperature sensor, wherein the electronic controller comprises a heating control module for controlling the temperature of the gas-selective electrode unit at a constant set temperature of at least 30°C, more preferably in the range of 35°C to 45°C. The reliability and the accuracy of the ammonium concentration measurements in the ammonium analyzer arrangement are highly dependent on the temperature of the electrolyte liquid, of the calibration liquid and of the sample liquid. Therefore, a constant temperature of the measurement unit is essential. As a side effect, the constant temperature of the measurement unit also results in a relatively constant evaporation of the electrolyte liquid in the electrolyte chamber housing so that other aging effects of the electrolyte liquid are foreseeable as well. This results in the fact that one single calibration/exhaustion graph can be sufficient to predict the electrolyte liquid volume at the basis of the calibration liquid measurement values.

Preferably the calibration/exhaustion graph shows the electrolyte liquid volume in the electrolyte chamber housing versus the difference between an initial calibration measurement value and the actual calibration measurement value. The initial calibration measurement value is the very first calibration measurement value after a new or a refilled gas-selective electrode unit has been put into operation. More preferably, the initial calibration measurement value can be an average value of two or more consecutive initial calibration measurements.

Preferably, the calibration liquid has an ammonium standard concentration of at least 5 mg/l. The higher the ammonium standard concentration of the calibration liquid is, the more reliable is the determination of the actual volume of the electrolyte liquid.

Preferably, the measurement signal evaluation module generates an ammonium concentration value at the basis of the actual calibration liquid measurement value.

Preferably, the ammonium analyzer arrangement is provided with a reagent liquid tank comprising a reagent liquid of which a suitable volume is pumped to the sample chamber for reacting with the sample liquid or with the calibration liquid in the sample chamber.

One embodiment of the invention is described with reference to the enclosed drawings, wherein:
Figure 1 schematically shows an ammonium analyzer arrangement with a gas-selective electrode unit and an electronic controller comprising a calibration module with an electrolyte exhaustion determination module comprising an exhaustion-graph memory memorizing a calibration/exhaustion graph, and
figure 2 the calibration/exhaustion graph as memorized in the exhaustion-graph memory.

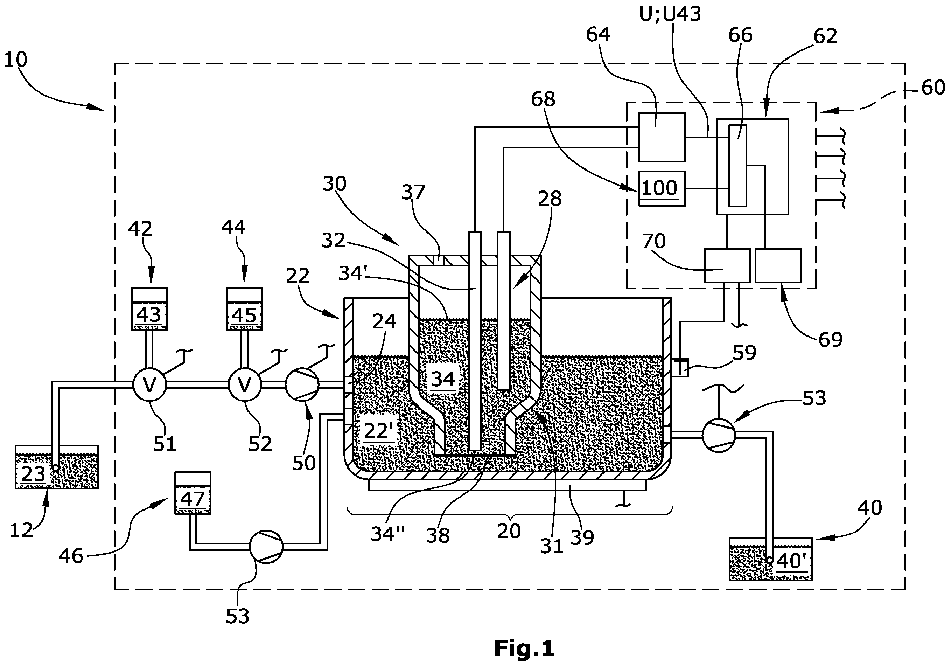

Figure 1 schematically shows a land-based ammonium analyzer arrangement 10 which is typically a process analyzer arrangement. The ammonium analyzer arrangement 10 determines the concentration of ammonium in a water basin 12 which could be a part of a water treatment plant. The water basin 12 is filled with a sample liquid 23 which can be pumped by a liquid pump 50 from the water basin 12 to a measurement unit 20.

The ammonium analyzer arrangement 10 is provided with a first calibration liquid container 42 with the first calibration liquid 43 and is provided with a second calibration liquid container 44 with a second calibration liquid 45. The first calibration liquid 43 in the first calibration liquid container 42 has an ammonium concentration of, for example, 10 mg/l, and the second calibration liquid 45 has a relatively low ammonium concentration of, for example 1.0 mg/l.

The ammonium analyzer arrangement 10 is provided with two 3-way valves 51, 52 for selectively leading the sample liquid 23, the first calibration liquid 43 or the second calibration liquid 45 via the liquid line between the water basin 12 and a sample chamber 22 of the measurement unit 20 and the liquid pump 50 to a sample inlet opening 24 of the sample chamber 22. The ammonium analyzer arrangement 10 is also provided with a waste liquid pump 53 four pumping the chamber liquid 22' in the sample chamber 22 after a measurement action to a wastewater tank 40 where the wastewater 40' is accumulated.

The ammonium analyzer arrangement 10 is provided with a reagent tank 46 comprising a reagent liquid 47 which can be pumped by a reagent pump 53 to the measurement unit 20. The reagent liquid 47 preferably is an alkaline solution, for example NaOH.

The measurement unit 20 substantially comprises the sample chamber 22 filled with the chamber liquid 22' which can be, in addition to the reagent liquid 47, the sample liquid 23 or can be one of the calibration liquids 43, 45, comprises a reference electrode element 28 which can be of any suitable type, and comprises a gas-selective electrode unit 30. Both electrode units 28, 30 are, at least in part, immersed into the chamber liquid 22'.

The gas-selective electrode unit 30 comprises an electrolyte chamber housing 31 made of glass or plastic, which is filled with a suitable electrolyte liquid 34 having an electrolyte liquid level 34'. A new or refilled gas-selective electrolyte unit 30 comprises 11 ml of the electrolyte liquid 34. The electrolyte liquid 34 can have, for example, a concentration of 100 mmol/l ammonium chloride, or can have, for a lower measurement range, a concentration of 2.0 mmol/l ammonium chloride.

The electrolyte chamber housing 31 is provided with a venting opening 37 at the top of the electrolyte chamber housing 31 through which the electrolyte liquid 34 constantly evaporates. The electrolyte chamber housing 31 comprises at its bottom a gas-sensitive membrane 38 made of PTFE and separating the electrolyte liquid 34 within the electrolyte chamber housing 31 from the chamber liquid 22' in the sample chamber 22.

The gas-selective electrode unit 30 is also provided with a measurement electrode element 32 being in direct contact with a thin electrolyte liquid layer 34" of a small volume of, for example, 10 µl of the electrolyte liquid 34, which electrolyte liquid layer 34" is provided between the distal sensor surface of the measurement electrode element 32 and the gas-sensitive membrane 38.

The measurement unit 20 is provided with an electrical heating 39 and a temperature sensor 59 which both are electrically connected to a heating control module 70 for indirectly controlling and keeping the temperature of the gas-selective electrode unit 30 at a constant temperature T of 45°C.

The electronic controller 60 comprises a measurement signal evaluation module 64 which is electrically connected to the reference electrode element 28 and to the gas-selective electrode unit 30 for generating a measurement value U being the difference of the voltage of the reference electrode element 28 and of the gas-selective electrode unit 30. The electronic controller 60 generates an ammonium concentration value at the basis of the measurement value U.

The electronic controller 60 comprises a calibration module 66, an electrolyte exhaustion determination volume 62, an exhaustion-graph-memory 68 memorizing a calibration/exhaustion graph 100 and a monitor 69.

The calibration module 66 controls and initiates a calibration run, for example, once per day. When the calibration module 66 starts a calibration run, the first calibration liquid 43 is pumped into the sample chamber 22, and a suitable volume of the reagent liquid 47 is pumped to the sample chamber 22, as well. The dissolved ammonium ions will then be converted to ammonia gas which passes the gas-sensitive membrane 38 and causes an electric potential change at the measurement electrode element 32.

After some minutes, the measurement signal evaluation module 64 is instructed to generate a calibration liquid measurement value U43 which is an electric voltage value being the voltage difference of the electric potentials at the reference electrode element 28 and at the measurement electrode element 32.

After that, a calibration run is initiated with the second calibration liquid 45 which results in a second calibration liquid measurement value U45. Both calibration liquid measurement values U43, U45 are memorized and used for the ammonium measurements and determinations of the following 24 hours.

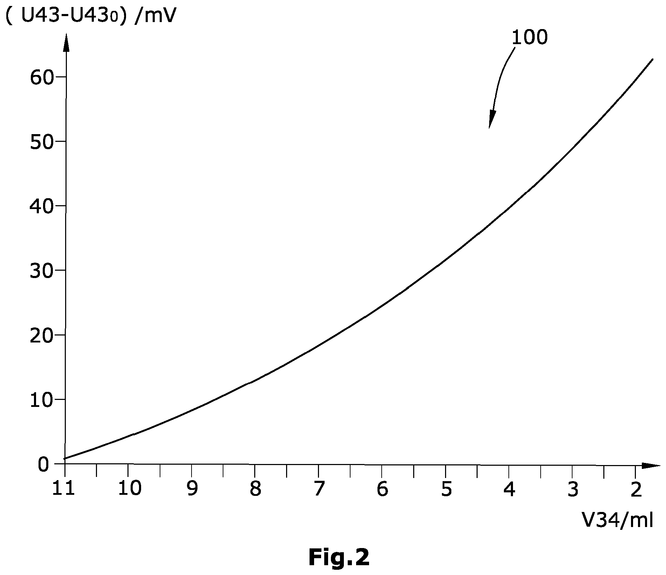

The memorized first calibration liquid measurement value U43 is also used by the electrolyte exhaustion determination module 62 to determine the volume V34 of the electrolyte liquid 34 in the electrolyte chamber housing 31. The electrolyte exhaustion determination module 62 determines the corresponding electrolyte liquid volume V34 from the calibration/exhaustion graph 100 memorized in the exhaustion-graph-memory 68 and shown in figure 2.

The actual electrolyte liquid volume V34 is displayed at the display 69. If the actual electrolyte liquid volume V34 is going to be exhausted, the electrolyte exhaustion determination module 62 causes the display 69 to present a maintenance recommendation. A relevant exhaustion of the electrolyte liquid volume V34 is reached at, for example, 3.0 ml.

## Claims

1. An ammonium analyzer arrangement (10) with an electronic controller (60) and a measurement unit (20), the measurement unit (20) comprising
a sample chamber (22) filled with a chamber liquid (22') being a sample liquid (23) or a calibration liquid (43), and
a gas-selective electrode unit (30) with an electrolyte chamber housing (31) filled with an electrolyte liquid (34),
with a reference electrode element (28) being in direct contact with the electrolyte liquid (34),
with a gas-sensitive membrane (38) separating the electrolyte liquid (34) from the chamber liquid (22'),
with a measurement electrode element (32) being in direct contact with the electrolyte liquid (34), and
with a venting opening (37) at the electrolyte chamber housing (31), wherein a calibration liquid container (42) with a calibration liquid (43) is provided,
wherein the electronic controller (60) comprises a measurement signal evaluation module (64) being connected to the reference electrode element (28) and to the measurement electrode element (32) configured to generate a measurement value (U),
wherein the electronic controller (60) comprises a calibration module configured (66) to generate a calibration liquid measurement value (U43) when the sample chamber (22) is filled with the calibration liquid (43),
wherein an electrolyte exhaustion determination module (62) comprising an exhaustion-graph-memory (68) memorizing a calibration/exhaustion graph (100) is provided,
wherein the electrolyte exhaustion determination module (62) is in signal- connection with the calibration module (66), and
wherein the electrolyte exhaustion determination module (62) configured to determine the actual volume (V34) of the electrolyte liquid (34) at the basis of the calibration measurement value (U43) and the calibration/exhaustion graph (100).

2. The ammonium analyzer arrangement (10) according to claim 1, wherein the measurement unit (20) is provided with a heating (39) and a temperature sensor (59), and wherein the electronic controller (60) comprises a heating control module (70) configured to controll the temperature of the gas-selective electrode unit (30) at a constant set temperature T of at least 30°C.

3. The ammonium analyzer arrangement (10) according to one of the preceding claims, wherein the gas-sensitive membrane (38) is made of PTFE.

4. The ammonium analyzer arrangement (10) according to one of the preceding claims, wherein the calibration/exhaustion graph (100) shows the electrolyte liquid volume (V34) versus the difference between an initial calibration measurement value (U43₀) and the actual calibration measurement value (U43).

5. The ammonium analyzer arrangement (10) according to one of the preceding claims, wherein the measurement signal evaluation module (64) configured to generate an ammonium concentration value at the basis of the actual calibration liquid measurement value (U43).

6. The ammonium analyzer arrangement (10) according to one of the preceding claims, wherein the calibration liquid (43) has an ammonium standard concentration of at least 5 mg/I.

7. A method for determining the actual volume (V34) of an electrolyte liquid (34) in an ammonium analyzer arrangement (10) with the features of one of the preceding claims, with the method steps:
filling the sample chamber (22) with the calibration liquid (43),
generating a calibration measurement value (U43), and
determining the electrolyte liquid volume (V34) by means of the calibration/exhaustion graph (100).

## Patentansprüche

1. Eine Ammonium-Analysatoranordnung (10) mit einer elektronischen Steuerung (60) und einer Messeinheit (20), wobei die Messeinheit (20) umfasst
eine Probenkammer (22), die mit einer Kammerflüssigkeit (22') gefüllt ist, die eine Probenflüssigkeit (23) oder eine Kalibrierflüssigkeit (43) ist, und
eine gasselektive Elektrodeneinheit (30) mit einem Elektrolytkammer-Gehäuse (31), das mit einer Elektrolytflüssigkeit (34) gefüllt ist,
wobei ein Referenzelektroden-Element (28) in direktem Kontakt mit der Elektrolytflüssigkeit (34) steht,
wobei eine gassensitive Membran (38) die Elektrolytflüssigkeit (34) von der Kammerflüssigkeit (22') trennt,
wobei ein Messelektroden-Element (32) in direktem Kontakt mit der Elektrolytflüssigkeit (34) steht, und
wobei eine Entlüftungsöffnung (37) an dem Elektrolytkammer-Gehäuse (31) vorgesehen ist,
wobei ein Kalibrierflüssigkeits-Behälter (42) mit einer Kalibrierflüssigkeit (43) vorgesehen ist,
wobei die elektronische Steuerung (60) ein Messsignal-Auswertemodul (64) umfasst, das mit dem Referenzelektroden-Element (28) und mit dem Messelektroden-Element (32) verbunden ist und zum Erzeugen eines Messwerts (U) ausgebildet ist,
wobei die elektronische Steuerung (60) ein Kalibriermodul (66) umfasst, das zum Erzeugen eines Kalibrierflüssigkeits-Messwerts (U43) ausgebildet ist, wenn die Probenkammer (22) mit der Kalibrierflüssigkeit (43) gefüllt ist,
wobei ein Elektrolyt-Erschöpfungs-Bestimmungsmodul (62) vorgesehen ist, das einen Erschöpfungs-Kurven-Speicher (68) umfasst, der einen Kalibrier-/Erschöpfungs-Kurve (100) speichert,
wobei das Elektrolyt-Erschöpfungs-Bestimmungsmodul (62) mit dem Kalibriermodul (66) in Signalverbindung steht, und
wobei das Elektrolyt-Erschöpfungs-Bestimmungsmodul (62) zum Bestimmen des tatsächlichen Volumens (V34) der Elektrolytflüssigkeit (34) auf der Basis des Kalibriermesswerts (U43) und der Kalibrier-/Erschöpfungs-Kurve (100) ausgebildet ist.

2. Die Ammonium-Analysatoranordnung (10) nach Anspruch 1, wobei die Messeinheit (20) mit einer Heizung (39) und einem Temperatursensor (59) versehen ist, und wobei die elektronische Steuerung (60) ein Heizungssteuermodul (70) umfasst, das zum Regeln der Temperatur der gasselektiven Elektrodeneinheit (30) auf eine konstante Solltemperatur T von mindestens 30 °C ausgebildet ist.

3. Die Ammonium-Analysatoranordnung (10) nach einem der vorhergehenden Ansprüche, wobei die gassensitive Membran (38) aus PTFE hergestellt ist.

4. Die Ammonium-Analysatoranordnung (10) nach einem der vorhergehenden Ansprüche, wobei die Kalibrier-/Erschöpfungs-Kurve (100) das Elektrolytflüssigkeits-Volumen (V34) gegenüber der Differenz zwischen einem anfänglichen Kalibriermesswert (U43₀) und dem tatsächlichen Kalibriermesswert (U43) zeigt.

5. Die Ammonium-Analysatoranordnung (10) nach einem der vorhergehenden Ansprüche, wobei das Messsignal-Auswertemodul (64) zum Erzeugen eines Ammonium-Konzentrationswerts auf der Basis des tatsächlichen Kalibrierflüssigkeits-Messwerts (U43) ausgebildet ist.

6. Die Ammonium-Analysatoranordnung (10) nach einem der vorhergehenden Ansprüche, wobei die Kalibrierflüssigkeit (43) eine Ammonium-Standardkonzentration von mindestens 5 mg/l aufweist.

7. Ein Verfahren zum Bestimmen des tatsächlichen Volumens (V34) einer Elektrolytflüssigkeit (34) in einer Ammonium-Analysatoranordnung (10) mit den Merkmalen eines der vorhergehenden Ansprüche, mit den Verfahrensschritten:
Füllen der Probenkammer (22) mit der Kalibrierflüssigkeit (43),
Erzeugen eines Kalibriermesswerts (U43), und
Bestimmen des Elektrolytflüssigkeits-Volumens (V34) mittels der Kalibrier-/Erschöpfungs-Kurve (100).

## Revendications

1. Un agencement d'analyseur d'ammonium (10) avec un dispositif de commande électronique (60) et une unité de mesure (20), l'unité de mesure (20) comprenant
une chambre d'échantillon (22) remplie d'un liquide de chambre (22') qui est un liquide d'échantillon (23) ou un liquide d'étalonnage (43), et
une unité d'électrode sélective au gaz (30) avec un boîtier de chambre d'électrolyte (31) rempli d'un liquide d'électrolyte (34),
avec un élément d'électrode de référence (28) étant en contact direct avec le liquide d'électrolyte (34),
avec une membrane sensible au gaz (38) séparant le liquide d'électrolyte (34) du liquide de chambre (22'),
avec un élément d'électrode de mesure (32) étant en contact direct avec le liquide d'électrolyte (34), et
avec une ouverture de ventilation (37) au niveau du boîtier de chambre d'électrolyte (31),
dans lequel un récipient de liquide d'étalonnage (42) avec un liquide d'étalonnage (43) est prévu,
dans lequel le dispositif de commande électronique (60) comprend un module d'évaluation de signal de mesure (64) étant relié à l'élément d'électrode de référence (28) et à l'élément d'électrode de mesure (32) configuré pour générer une valeur de mesure (U),
dans lequel le dispositif de commande électronique (60) comprend un module d'étalonnage configuré (66) pour générer une valeur de mesure de liquide d'étalonnage (U43) lorsque la chambre d'échantillon (22) est remplie du liquide d'étalonnage (43),
dans lequel un module de détermination d'épuisement d'électrolyte (62) comprenant une mémoire de graphique d'épuisement (68) mémorisant un graphique d'étalonnage/d'épuisement (100) est prévu,
dans lequel le module de détermination d'épuisement d'électrolyte (62) est en liaison de signal avec le module d'étalonnage (66), et
dans lequel le module de détermination d'épuisement d'électrolyte (62) est configuré pour déterminer le volume réel (V34) du liquide d'électrolyte (34) sur la base de la valeur de mesure d'étalonnage (U43) et du graphique d'étalonnage/d'épuisement (100).

2. L'agencement d'analyseur d'ammonium (10) selon la revendication 1, dans lequel l'unité de mesure (20) est pourvue d'un chauffage (39) et d'un capteur de température (59), et dans lequel le dispositif de commande électronique (60) comprend un module de commande de chauffage (70) configuré pour commander la température de l'unité d'électrode sélective de gaz (30) à une température de consigne constante T d'au moins 30 °C.

3. L'agencement d'analyseur d'ammonium (10) selon l'une des revendications précédentes, dans lequel la membrane sensible au gaz (38) est constituée de PTFE.

4. L'agencement d'analyseur d'ammonium (10) selon l'une des revendications précédentes, dans lequel le graphique d'étalonnage/d'épuisement (100) montre le volume de liquide d'électrolyte (V34) par rapport à la différence entre une valeur de mesure d'étalonnage initiale (U43₀) et la valeur de mesure d'étalonnage réelle (U43).

5. L'agencement d'analyseur d'ammonium (10) selon l'une des revendications précédentes, dans lequel le module d'évaluation de signal de mesure (64) est configuré pour générer une valeur de concentration d'ammonium sur la base de la valeur de mesure de liquide d'étalonnage réelle (U43).

6. L'agencement d'analyseur d'ammonium (10) selon l'une des revendications précédentes, dans lequel le liquide d'étalonnage (43) a une concentration standard d'ammonium d'au moins 5 mg/l.

7. Un procédé de détermination du volume réel (V34) d'un liquide d'électrolyte (34) dans un agencement d'analyseur d'ammonium (10) avec les caractéristiques de l'une des revendications précédentes, comprenant les étapes de procédé :
remplissage de la chambre d'échantillon (22) avec le liquide d'étalonnage (43),
génération d'une valeur de mesure d'étalonnage (U43), et
détermination du volume de liquide d'électrolyte (V34) a moyen du graphique d'étalonnage/d'épuisement (100).
